Europäisches Patentamt

(19) European Patent Office

Office européen des brevets

(11) **EP 0 599 955 B1**

(12) **EUROPEAN PATENT SPECIFICATION**

(45) Date of publication and mention
of the grant of the patent:
**11.11.1998 Bulletin 1998/46**

(21) Application number: **92918013.1**

(22) Date of filing: **11.08.1992**

(51) Int. Cl.$^6$: **A61B 17/36**

(86) International application number:
**PCT/US92/06693**

(87) International publication number:
**WO 93/03678 (04.03.1993 Gazette 1993/06)**

(54) **LATERALLY REFLECTING TIP FOR LASER TRANSMITTING FIBER**

SEITLICH REFLEKTIERENDE SPITZE FÜR LASERÜBERTRAGENDE FASER

EMBOUT A REFLEXION LATERALE POUR FIBRE TRANSMETTANT UN FAISCEAU LASER

(84) Designated Contracting States:
**DE FR GB IT**

(30) Priority: **16.08.1991 US 746418**
**16.08.1991 US 746475**

(43) Date of publication of application:
**08.06.1994 Bulletin 1994/23**

(73) Proprietor: **MYRIADLASE, INC.**
**Forest Hill, TX 76140-1505 (US)**

(72) Inventors:
• **JUDY, Millard, M.**
  **Dallas, TX 75214 (US)**
• **MATTHEWS, James, L.**
  **Dallas, TX 75214 (US)**
• **GARDETTO, William, W.**
  **Bedford, TX 76021 (US)**

(74) Representative:
**Allen, William Guy Fairfax et al**
**J.A. KEMP & CO.**
**14 South Square**
**Gray's Inn**
**London WC1R 5LX (GB)**

(56) References cited:
**EP-A- 0 441 606**          **WO-A-89/00408**
**WO-A-89/11834**           **US-A- 2 663 254**
**US-A- 2 891 425**          **US-A- 3 243 165**
**US-A- 3 321 863**          **US-A- 3 919 524**
**US-A- 4 123 143**          **US-A- 4 467 171**
**US-A- 4 541 139**          **US-A- 4 592 353**
**US-A- 4 819 632**          **US-A- 5 029 588**
**US-A- 5 041 121**

## Description

The present invention relates generally to devices for use in laterally reflecting light energy from a flexible elongated light transmitting fiber, and more particularly, but not by way of limitation, to such devices designed for use with laser light transmitting fibers.

One developing area of medical technology involves the application of light energy, typically laser light energy, to a site in the patient's body to alter, remove or destroy tissue in the patient's body. This may be done with bare fibers or with fibers having a metal tip on the distal end. Such tips typically absorb all or part of the laser energy so that the tip itself is heated to a clinically significant extent whereby the tissue is at least partially treated by heat conduction from the heated tip which is placed in contact with the tissue. The tips also may have apertures for directing a portion of the laser light either directly or by reflection upon the tissue to be treated.

Many medical procedures utilizing such tips are more easily accomplished if the laser energy is reflected laterally from the longitudinal axis of the fiber so that it can be directed upon tissue which would be difficult to treat with light emitted axially from the fiber.

WO-A-89/11834 discloses an operating assembly for use in an operating environment comprising: a flexible elongated light transmitting fibre having a distal end and a distal fibre end defined therein; and a light reflecting tip attached to said distal end portion of said fibre said tip including a reflecting mirror surface so arranged and constructed as to laterally reflect light exiting said distal fibre end, said tip having a lateral passageway defined therein.

The present invention provides a number of improvements in the construction of tips of this type generally described.

The assembly of the invention is characterised by the features as defined in the second part of claim 1.

The lateral passageway through the body provides a flushing means for permitting surrounding fluid to flow through the lateral passageway across the reflecting mirror surface to reduce collection of contaminants on the reflecting mirror surface.

In a preferred embodiment, the tip is easily and securely attached to the flexible elongated light transmitting fiber. In this embodiment the assembly includes an elongated body having a distal head portion and having a plurality of flexible legs extending proximally from the head portion. A clamping means is operably associated with the body for urging the legs radially inward to clamp the distal end portion of the fiber between the legs. This is preferably provided by a two-piece tip wherein the first piece includes the flexible legs which have outer surfaces thereon which define a conically tapered outer surface of the first piece. The second piece includes a sleeve slidably received about the legs. The sleeve includes a conically tapered inner surface complementary to the conically tapered outer surface of the legs causes the legs to be biased radially inward to clamp the distal end portion of the fiber therebetween. This mounting arrangement is useful on a variety of tip designs and is not limited to laterally transmitting tips.

In a preferred construction of the invention, a laterally reflecting tip is provided which reflects substantially all incident laser light energy laterally outward through an aperture so that there is no clinically significant heating of the tip. The tip includes an elongated thin cylindrical body having a fiber receiving longitudinal opening therein for receiving the distal end portion of the fiber. A reflecting mirror surface means is defined on the body for reflecting light from the fiber laterally outward through the lateral passageway.

The light reflecting mirror surface may be formed by providing a gold reflecting surface on the tip, and coining the gold reflecting surface to provide a superior reflecting surface.

Alternatively the light reflecting mirror surface may be formed from a first metal other than gold. An underlying gold layer is then sputter deposited on the body. Finally an outer gold sheet is diffusion bonded under external pressure at elevated temperature to the underlying gold layer, with the outer surface of the outer gold sheet defining the light reflecting mirror surface. Preferably this light reflecting mirror surface is then coined to provide a superior reflecting surface.

In order that the present invention may more readily be understood, the following description is given, merely by way of example, reference being made to the accompanying drawings in which:-

FIG. 1 is a plan view of the light reflecting tip assembled with a flexible elongated light transmitting fiber.

FIG. 2 is an elevation sectioned view taken along line 2-2 of FIG. 1.

FIG. 3 is a plan view similar to FIG. 1 of the elongated body of the two-piece tip assembly of FIGS. 1 and 2.

FIG. 4 is an elevation sectioned view of the body of FIG. 3 taken along line 4-4.

FIG. 5 is a right end view of the body of FIG. 4.

FIG. 6 is an elevation sectioned view of the sleeve piece of the two-piece tip assembly of FIGS. 1 and 2.

FIG. 7 is a schematic illustration similar to FIG. 2 showing the beam spread of a beam reflected from a flat mirror.

FIG. 8 is a view similar to FIG. 7 showing the beam spread of a beam reflected from a concave mirror.

FIG. 9 is a view similar to FIG. 7 showing the beam spread of a beam reflected from a convex mirror.

FIG. 10 is a schematic illustration of the laser and endoscope with which the fiber and tip are used.

FIG. 11 is an enlarged sectioned view of a portion of the endoscopic tube of FIG. 10, illustrating the passage of the fiber and tip through a curved endoscopic tube.

FIGS. 12A and B and 13A and B schematically represent certain tests which were run to determine reflective efficiency of the tips.

FIGS. 14 and 15 are graphic representations of the spreading of the light beam energy by a flat reflective mirror surface.

FIGS. 16 and 17 schematically illustrate the methodology of an example test which shows that there is no clinically significant heating of the tip.

Detailed Description Of The Preferred Embodiments

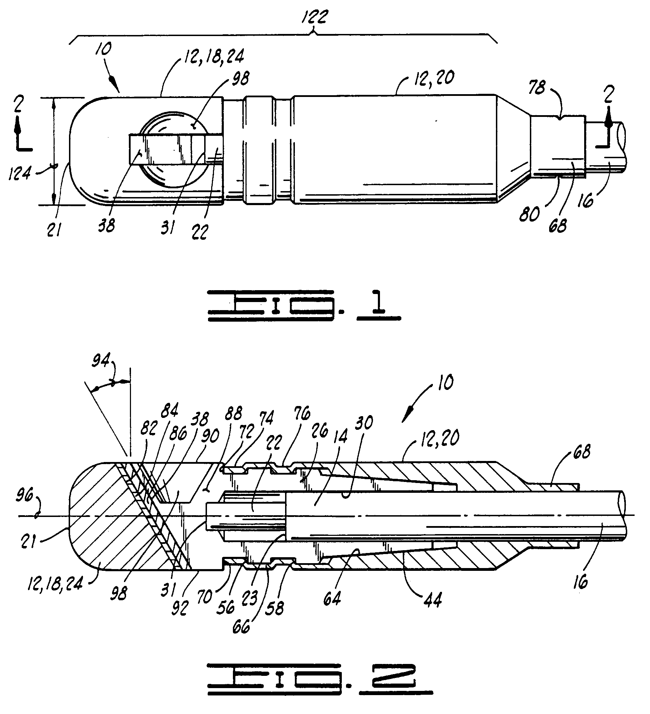

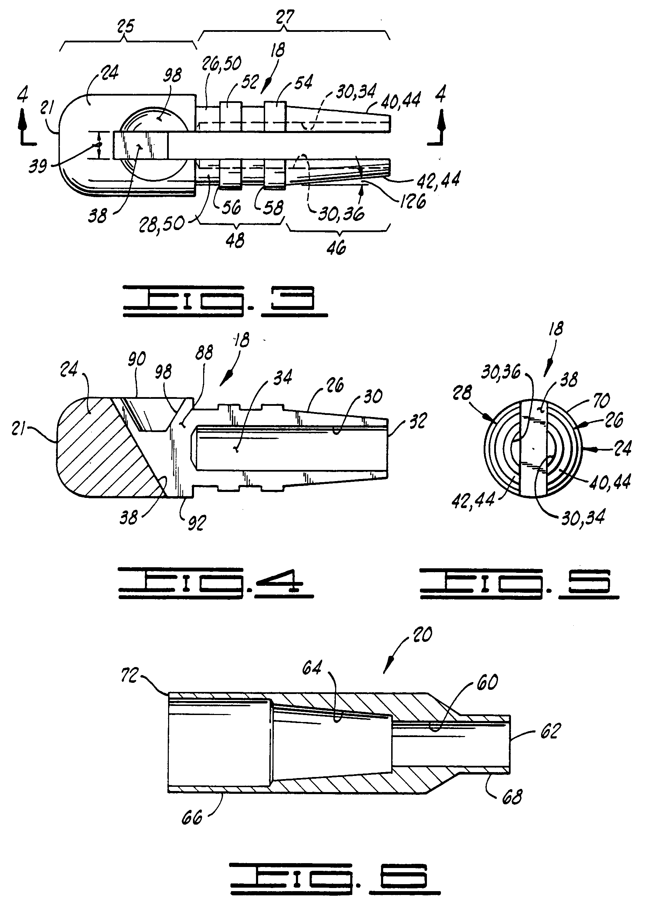

Referring now to the drawings, and particularly to FIGS. 1 and 2, a tip and fiber assembly is thereshown and generally designated by the numeral 10. The assembly 10 includes a two-piece light reflecting tip 12 mounted upon a distal end portion 14 of a flexible elongated light transmitting fiber 16.

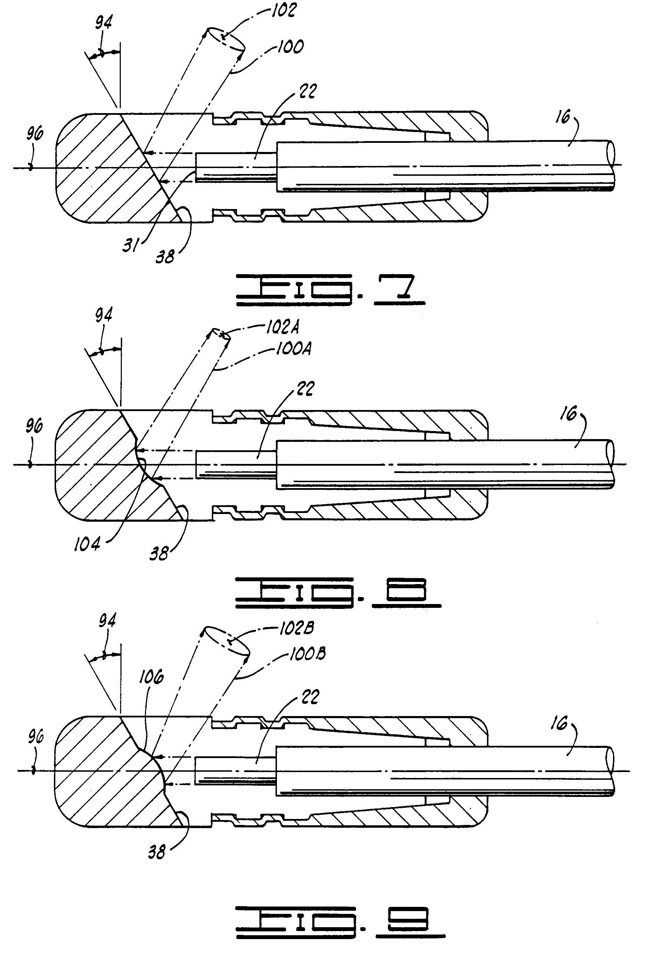

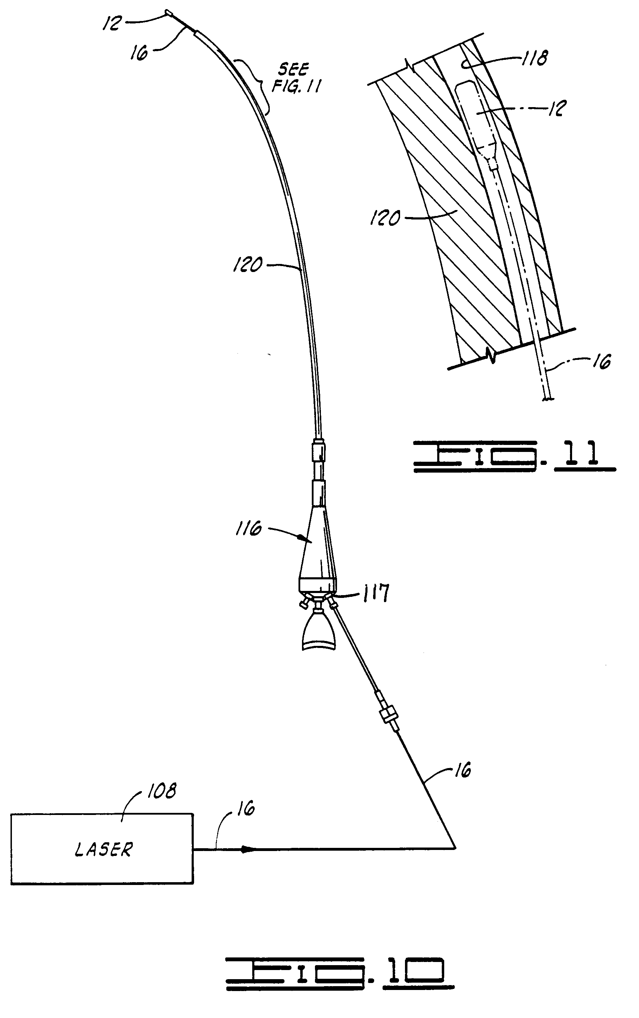

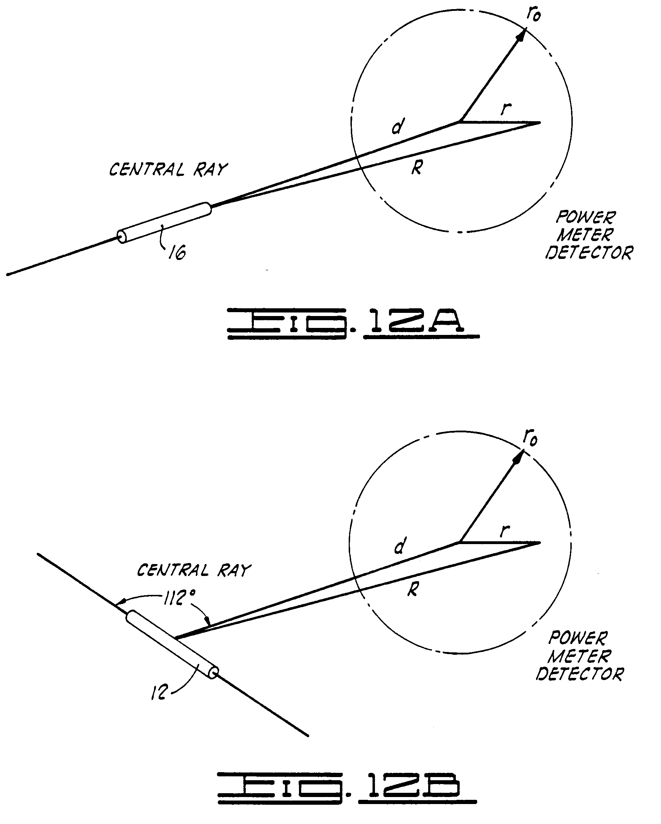

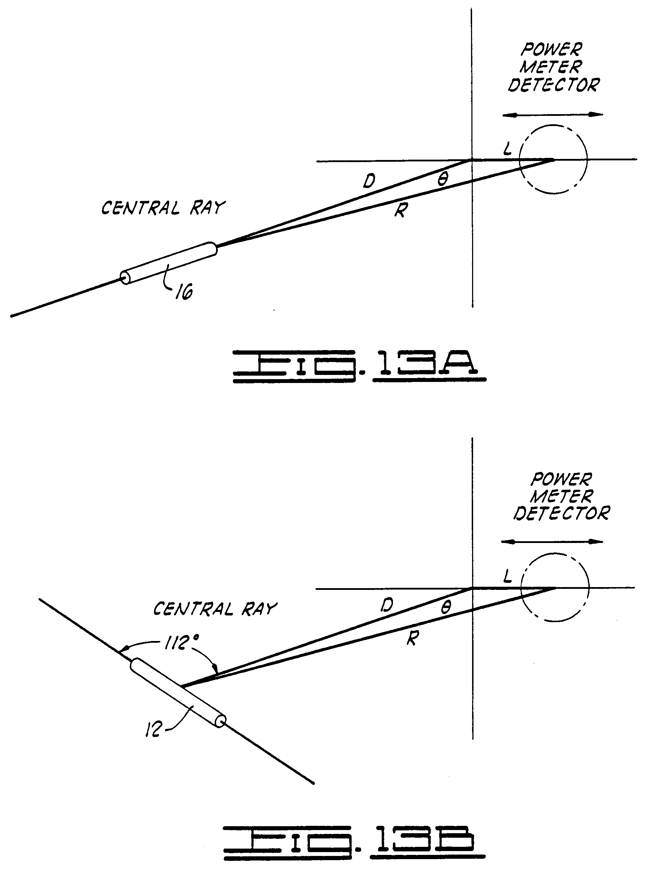

The two-piece tip 12 includes an elongated body 18 which is best shown in isolation in FIGS. 3-5 and a clamping sleeve 20 which is illustrated in isolation in FIG. 6.

The fiber 16 is a commercially available laser light transmitting fiber. Such fibers include a flexible elongated cylindrical core 22 which is surrounded by a cladding of lower optical refractive index material which is overlain with an outer protective jacket. As seen in FIG. 2, when assembled with the tip of the present invention, the outer cladding and jacket of the fiber 16 are preferably trimmed back to end at 23 with the fiber core 22 extending distally beyond the end 23 of the outer protective layers. This minimizes the possibility of burning of the outer protective layers due to laser energy reflected back from the mirror surface.

The elongated body 18 has a distal head portion 24 and has first and second flexible legs 26 and 28 extending proximally from the head portion 24. Although there is no critical line of distinction between the head portion 24 and the legs 26 and 28, the head portion 24 can generally be described as having a length 25 as seen in FIG. 3, and the legs 26 and 28 can generally be described as having a length 27. Head portion 24 has a squared-off flat end 21.

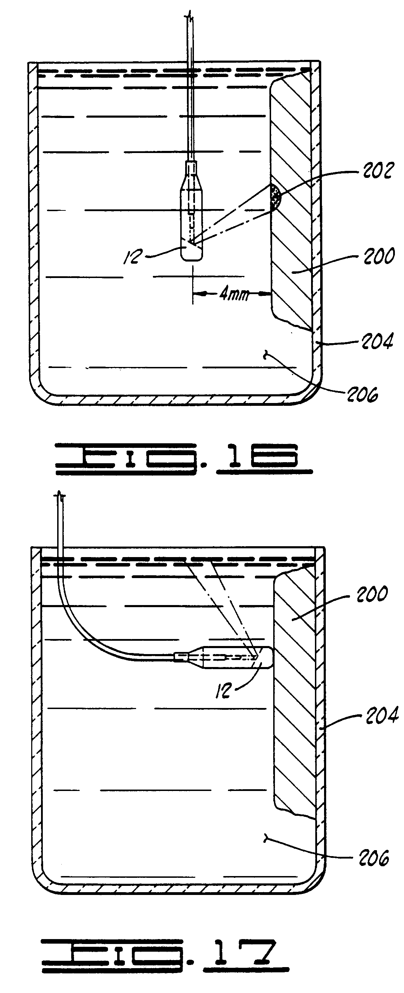

As used herein the terms distal and proximal are used in relation to the terminology generally used for the fiber 16 with the distal end of fiber 16 being indicated at 31 in FIG. 2, and with the proximal end of the fiber 16 being the other end which typically would be attached to the laser light source 108 (see FIG. 10). Thus the distal and proximal directions or relative orientations are provided for the tip 12 used in association with the fiber 16.

The clamping sleeve 20 can be more generally described as a clamping means 20 operably associated with the body 18 for urging the legs 26 and 28 radially inward to clamp the distal end portion 14 of fiber 16 therebetween in a manner further described below.

As best seen in FIG. 4, a blind bore 30 is drilled into the proximal end 32 of body 18 thus defining partially cylindrical inner surfaces 34 and 36 on legs 26 and 28, respectively, which surfaces 34 and 36 define a body bore means 30 for closely receiving and clamping the distal end portion 14 of the fiber 16 therebetween.

The body 18 can be described as having a plurality of legs, which in the preferred embodiment are two and only two legs 26 and 28. The head portion 24 has a reflecting mirror surface means 38 defined thereon for reflecting light emitted from the distal end 31 of fiber 16 laterally outward. The body bore means 30 terminates short of the reflecting mirror surface 38.

The legs 26 and 28 have proximal conically tapered outer surface portions 40 and 42, respectively, which collectively define a conically tapered outer body surface 44 extending over a length 46.

A distal portion 48 of legs 26 and 28 defines a generally cylindrical outer surface 50 having first and second annular radially outward extending flanges 52 and 54 each of which has a distally facing shoulder 56 and 58, respectively, defined thereon.

The body 18 is preferably formed from a cylindrical stainless steel blank which is machined to have the appearance shown in FIGS. 3 and 4. The lateral passageway 88 and legs 26 and 28 are partially formed by milling a slot having the width 39 as seen in FIG. 3 into the cylindrical blank. As further described below, the body 18 may also be formed of solid gold in which case it will be cast in the shape shown in FIGS. 3 and 4.

The clamping sleeve 20 which is shown in its unassembled state in FIG. 6, is designed to be slidably received about the legs 26 and 28. Clamping sleeve 20 has an axial sleeve bore 60 defined through its proximal end 62 for closely receiving the fiber 16. Clamping sleeve 20 further includes a tapered conical sleeve counterbore 64 complementary to the conically tapered outer body surface 44 so that when the clamping sleeve 20 slides in a distal direction over the legs 26 and 28, engagement of the tapered conical sleeve counterbore 64 with the conically tapered outer body surface 44, causes the legs 26 and 28 to be biased radially inwardly.

The clamping sleeve 20 further includes a thin cylindrical distal portion 66 and a thin cylindrical proximal portion 68 which as further described below are used in securing the two-piece tip assembly together and onto the fiber 16.

As best seen in FIG. 2, a body abutment surface 70, which is an annular shoulder, is defined on the head 24 of body 18. A sleeve abutment surface 72, which is the distal end of sleeve 20, is defined on the clamping sleeve 20 and is arranged to abut the body abutment surface 70 to limit sliding motion of the clamping sleeve 20 over the legs 26 and 28.

The tapered conical sleeve counterbore 64 and the conically tapered outer body surface 44 have an interference fit prior to abutment of the sleeve abutment surface 72 with the body abutment surface 70. Thus, with the fiber 16 in place as shown in FIG. 2, as the clamping sleeve 20 slides distally over the legs 26 and 28, the sliding interference fit between tapered surfaces 64 and 44 causes the legs 26 and 28 to be cammed radially inward thus clamping fiber 16 therebetween.

After the sleeve abutment surface 72 engages the body abutment surface 70, the clamping sleeve 20 is secured to the body 18 by forming first and second circumferentially rolled crimps 74 and 76 in the thin cylindrical distal portion 66 of clamping sleeve 20 so that the crimps 74 and 76 engage the annular shoulders 56 and 58 to prevent the clamping sleeve 20 from sliding backwards off the legs 26 and 28. Additionally, the thin cylindrical proximal portion 68 of clamping sleeve 20 is further secured to the fiber 16 by diametrically opposed staking crimps 78 and 80 as seen in FIG. 1.

It will be appreciated that the construction of the legs and clamping sleeve could be reversed so that the sleeve protrudes from the body with the legs being formed on a separate piece which slides into engagement with the sleeve. More generally, the tip 12 can be described as a two-piece tip including a first piece having a plurality of longitudinally extending legs and a second piece including a sleeve slidably received about the legs.

The abutment surfaces 70 and 72 can be more generally described as a limit means 70, 72 for limiting insertion of the legs 26 and 28 within the clamping sleeve 20 and thereby limiting a clamping force applied to the fiber 16 by the legs 26 and 28.

It is noted that the body 18 shown in FIG. 4 differs in one aspect from the body 18 illustrated in FIG. 2, namely in regard to the construction of the reflecting mirror surface means 38.

The reflecting mirror surface means 38 is preferably made of biocompatible 99.9+ percent gold. This can be accomplished in at least two ways. In FIG. 4, the entire body 18 is made from gold and thus all that need be done to complete the reflecting mirror surface means 38 is to treat the surface to make it as smooth and reflective as possible.

FIG. 2, on the other hand, illustrates a construction wherein the major portion of the body 18 is made of a first metal other than gold, preferably 316 stainless steel. An underlying mirror support surface 82 is defined on this first material. Then gold is laid over the underlying support surface in such a way as to form the gold reflecting mirror surface means 38. One preferred manner of doing this is to sputter deposit an underlying gold layer 84 onto the underlying mirror support surface 82, and then to diffusion bond a gold sheet 86 to the underlying gold layer 84.

The diffusion bonded gold sheet 86 preferably is formed from rectangular gold wire having a thickness of at least 0.36 mm (0.014 inches) thus providing a gold layer thick enough to prevent the chemical action of the surrounding saline medium from corroding completely through the gold layer during typical medical treatment procedures. The gold sheet has a width approximately equal to the width 39 of mirror surface 38.

Regardless of whether the solid gold body of FIG. 4 or the stainless steel body with overlying gold layers of FIG. 2 is used, the final treatment of the gold reflecting mirror surface means 38 is preferably formed by coining the surface. Coining is a mechanical process whereby a very hard, smooth member is pressed against the surface 38 to stamp it or coin it so that it too carries a smooth highly reflective impression.

As is further described below, the smooth, coined, gold reflecting mirror surface means 38 provides a means for reflecting substantially all of the light energy incident thereon from the fiber 16.

Alternatively, the mirror surface 38 could be made smooth by electro or mechanical polishing or by chemical etching.

In designing a tip for maximum reflection so that substantially all laser light energy incident upon the reflecting surface 38 is reflected, it is necessary to choose the reflecting material dependent upon the particular laser being utilized. Different reflective materials have more efficient reflective characteristics for different wavelengths of laser light. For example, when utilizing an Nd:YAG laser having a characteristic wavelength of 1064 or 1318 nanometers, or when using a Ho:YAG laser having a characteristic wavelength of 2100 nanometers, the reflective surface 38 is preferably made of gold.

On the other hand, for a frequency doubled Nd:YAG laser having characteristic wavelengths of 532 or 659 nanometers, a platinum surface 38 is preferred. Similarly, the platinum surface is preferred for an Alexandrite laser having a 680-800 nanometer tuning range, a Sapphire laser having a 680-800 nanometer tuning range, an Argon Ion laser emitting radiation with wavelengths in the 428-528 nanometer range, and for a dye laser having a 400-800 nanometer tuning range.

Gold and platinum are chosen as preferred mirror surfaces on the basis of their high optical reflectivities and their relative stabilities in a warm aqueous saline solution environment.

As is best seen in FIG. 2, the body 18 has a lateral passageway 88 defined diametrically therethrough which intersects the body bore means 30. The lateral passageway 88 has diametrically opposite first and second end openings 90 and 92, respectively. The lateral passageway 88 extends across and runs parallel to the reflecting mirror surface means 38, and thus due to the slope of the reflecting mirror surface means 38, the second end opening 92 is substantially smaller than the first end opening 90 of lateral passageway 88.

The reflecting mirror surface means 38 is set at an angle 94 to the longitudinal axis 96 of the fiber 16 and tip 12. Thus light exiting the distal end 31 of fiber core 22 falls axially (with a small divergence) onto the reflecting mirror surface means 38 and then is reflected laterally out the larger first end opening 90 of lateral passageway 88.

There is some angular diffusion of the light which reflects from the reflecting mirror surface means 38 so that it tends to diffuse into a conical beam, and the body 38 has a conical cavity 98 superimposed upon the larger first end opening 90 of lateral passageway 88 thus permitting the reflected beam to pass laterally outward without impinging upon any other portions of the tip 12 other than the reflecting mirror surface means 38.

The tip 12 is constructed for usage in any number of medical procedures wherein the tip is used to direct laser light onto human tissue to treat the same. The tip 12 is typically immersed in an aqueous saline solution during operation, but it will be appreciated that often the solution is contaminated with proteins from the surrounding body tissue. If these proteins are allowed to build up on the reflecting mirror surface means 38, it of course will significantly degrade the operation of the tip 12. The lateral passageway 88 extending completely laterally through the body 18 over the reflecting mirror surface means 38 provides a flushing means 88 for permitting surrounding fluid, typically saline solution, to flow in one of the first and second end openings 90 and 92, then across the reflecting mirror surface means 38, then out the other of the first and second end openings, thus tending to cleanse the reflecting mirror surface means 38 and to reduce collection of contaminants on the reflecting mirror surface means 38.

The flushing of saline solution through the lateral passageway 88 also aids in carrying away saline solution heated by laser light passing therethrough to prevent any significant heat buildup adjacent the tip 12. As further shown below, the tip 12 is designed for substantially complete reflection of the laser light energy incident upon mirror 38. There is no clinically significant heating of tip 12.

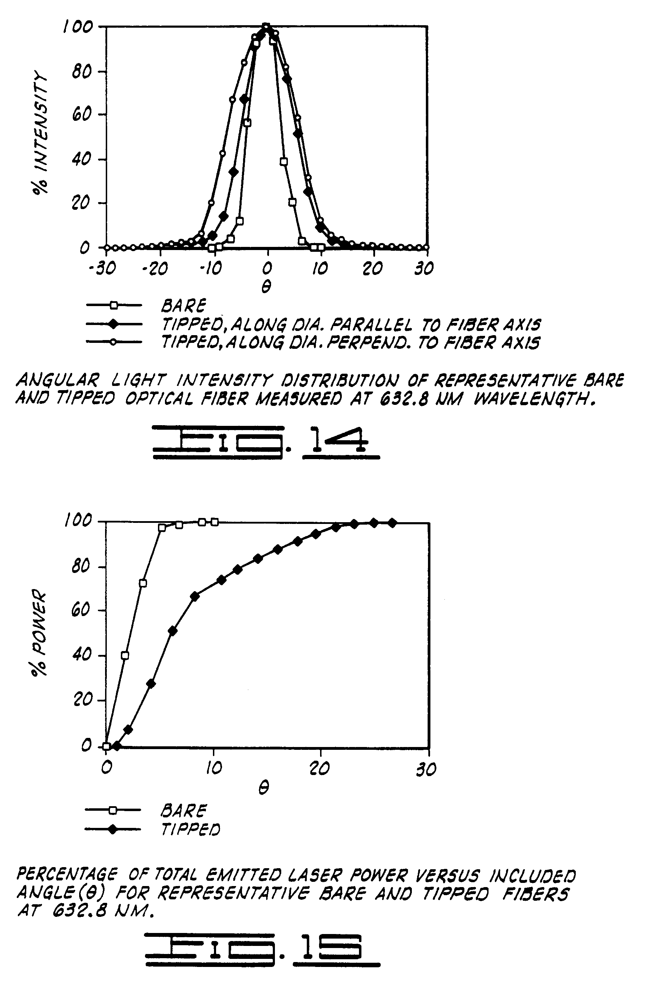

FIGS. 7-9 are somewhat simplified versions of FIG. 2 and they illustrate various modifications that can be made to the reflecting mirror surface means 38.

FIG. 7 illustrates the flat planar reflecting mirror surface means 38 previously described with regard to FIGS. 2 and 4. In a preferred embodiment the angle 94 of surface 38 is 37½°, and the distal end 31 of fiber core 22 is cut square, i.e., it defines a plane normal to the axis 96 of the fiber. With such an arrangement, and with a highly reflective coined gold reflecting mirror surface means 38, the reflected beam 100, the outer extremities of which are shown in phantom lines will diverge slightly from a cylindrical beam due to divergence upon exiting fiber core 22 and due to imperfect reflection of the mirrored surface 38 so as to form a reflected light spot 102. In a preferred embodiment the flat coined gold reflecting mirror surface means 38 of FIG. 7 is a means for providing a generally circular reflected light spot size 102 no greater than 3 mm diameter at a distance of 1.05 cm along the central ray of the deflected beam from the central axis 96 of the fiber 16.

It is noted that with the specified angle 94, the reflected beam will reflect somewhat in a proximal direction. This back reflection is a significant aid in directing the reflected beam to hard-to-reach portions of cavities within the human body. The angle of reflection of the beam can of course be modified as desired by modifying the angle 94 and the configuration of the reflecting surface 38. Also, the distal end 31 of the fiber core 22 can be cut at an angle which will also affect the dispersion and direction of the light beam emitted therefrom.

As best seen in FIG. 2, there is an axial clearance between the distal end 31 of fiber core 32 and the reflecting mirror surface means 38. This permits the lowermost reflected beam to exit through the lateral passageway 88 without impinging upon the fiber end 31 or any portion of the tip 12 other than the mirror 38.

Depending upon the type of treatment being conducted, it may be desirable for the reflected light spot 102 to be either more diffused or more concentrated. This can be accomplished by modifying the reflecting mirror surface means 38 as shown in FIGS. 8 or 9 to concentrate or disperse the beam, respectively.

In FIG. 8, the reflecting mirror surface means 38 has a concave portion 104 which is impinged by the beam from fiber core 22 thus concentrating the reflected beam 100A to form a smaller reflected beam spot 102A as compared to FIG. 7.

In FIG. 9, the reflecting mirror surface means 38 includes a convex portion 106 which causes the reflected beam 100B to be more dispersed and thus form a larger reflected beam spot 102B as compared to the apparatus of FIG. 7.

It will be appreciated that FIGS. 7-9 are only schematically illustrated and are not necessarily geometrically correct. The angles of the beams have been somewhat exaggerated to illustrate the difference between the various embodiments.

FIG. 10 schematically illustrates the various apparatus with which the fiber 16 and tip 12 are typically used. The proximal end of the fiber 16 is connected to a laser 108 which produces the laser light which is transmitted through the fiber 16 and then reflected by tip 12. In the present disclosure the word light is used in its broad sense, meaning electromagnetic radiation which propagates through space and includes not only visible light, but also infrared, ultraviolet and microwave radiation. In the preferred embodiment, the light transmitted by fiber 16 is laser light produced by the laser 108.

The fiber 16 is typically utilized with a conventional endoscope 116, which may be rigid or flexible, and which carries the fiber 16 in a cylindrical passage 118 defined in an endoscopic tube 120. The endoscopic tube 120 may include a parallel optical viewing fiber (not shown) and parallel fluid flow conduits (not shown). Also, the cylindrical passage 118 may itself be utilized as a fluid flow passage to allow irrigating fluids to flow to the tip 12.

It will be appreciated that depending upon the medical procedure being performed, the endoscopic tube typically may need to pass through various curved cavities in the human body so as to reach the area to be treated. In such case a flexible endoscope will be used.

The tip 12 and fiber 16 which are assembled according to the present invention preferably are constructed so that they can pass through the cylindrical passage 118 of endoscopic tube 120 when the endoscopic tube 120 is contorted in the necessary fashion. Thus, the fiber 16 assembled with tip 12 must be capable of curving to pass through the curved cylindrical passage 118. Also, the fiber 16 and tip 12 must pass through the endoscope 116 itself. Typically the most severe curvature through which the fiber 16 and tip 12 must pass is the bend at connection 117 where the fiber 16 enters the endoscope 116 from a laterally offset position and then must pass into the tube 120. The radius of curvature of this bend on existing endoscopes is approximately 10 cm. This passage may have a diameter on existing endoscopes as small as 2.5 mm.

FIG. 11 is a schematic, segmented, cross-section portion of the endoscopic tube 120 in the curved area designated in FIG. 10. The fiber 16 and tip 12 are shown in phantom lines in FIG. 11 as they would appear when the tip 12 and fiber 16 are being run in a distal direction through the passage 118 so that they ultimately will extend from the distal end of endoscopic tube 120 as illustrated in FIG. 10.

A typical passage 118 of available endoscopic tubes 120 has a diameter of no greater than 2.5 mm. The preferred optical fiber described below has a design minimum bending radius of 1 cm which can easily conform to passage 118 so long as the tip 112 can fit through the curved passage. Since the most severe bend to be encountered is that at junction 117, the fiber 16 and tip 12 should be so constructed and dimensioned as to provide an assembly capable of passing through a curved cylindrical passage having a diameter of 2.5 mm and a radius of curvature of 10 cm.

Prior art tip constructions for laterally reflecting tips have been relatively bulky and have been incapable of passing through such a narrow curved passage. The present invention has solved this problem. In a preferred embodiment this is accomplished by constructing the tip 12 to have a length 122 (see FIG. 1) of 0.3543 inches (9.000 mm) and an outside diameter 124 of 0.0866 inches (2.200 mm). This tip is generally cylindrical in shape and is concentrically mounted upon the distal end portion 14 of fiber 16. Preferably the tip 12 has a maximum diameter 124 of no greater than 2.2 mm, and a length no greater than 1 cm.

The preferred light transmitting fiber 16 for use with the tip 12 having the dimensions just described is a conventional fused silica fiber. This fiber has a minimum bending radius of 1 cm. The silica core has a diameter of 600 microns and the fiber with cladding has an outside diameter of 1.02 mm (0.040 inch) which is clamped within the bore 30 of tip 12 which has a bore diameter of 1.02 mm (0.040 inches).

In this preferred embodiment the tapered outer surface 44 and tapered inner counterbore 64 have an angle of taper such as indicated at 126 in FIG. 3 in the range of from 4.2° to 4.8°.

Efficiency of Reflection

Measurements of reflective optical power and intensity distribution over the reflected beam were made on a group of nine fibers having tips 12 with 99.9% gold reflective surface formed as described above with regard to FIG. 2. Similar measurements were also made on a corresponding group of bare fibers of identical manufacture.

Specifically, measurements were made to assess and compare the reproducibility of values of reflected and emitted power and radiated intensity distribution within each group of optical fibers and to compare the efficiency of the reflection by the actual gold surface relative to the theoretically predicted value for an ideal gold surface.

Measurements to determine emitted and reflected radiation power were made using the set-up shown in FIGS. 12A and 12B. In the tip measurements the central ray of the reflected beam was deviated 112 degrees from the parent fiber axis, and impacted the circular detector surface at its center. The central ray of the emerging beam was aligned in similar fashion relative to the detector for measurements on bare fibers.

Measurements to determine the uniformity of reflected power from tipped fibers and of emitted power from the bare fibers respectively, were made at constant input using the Nd:YAG laser power settings of 5, 10 and 20 watts. The distance from either reflecting or emitting surface was kept at 7 centimeters. This was done to keep the fixed geometry for measurement at all power levels selected without bleaching or burning the blackened detector surface.

Experiments were also performed on three fibers randomly picked from the tipped fiber population in order to assess the efficiency or percentage of power reflected in comparison to the average emitted power of the bare fiber population. These measurements were made at 10 watts input Nd:YAG laser power delivered to each fiber and with the fiber end located 1 centimeter from the detector surface so as to collect sensibly all the reflected beam pattern. Collection of the entire beam with this geometry was established from the accompanying measurements (described below) of radiation intensity over the reflected beam.

Measurements of the uniformity of distribution of radiation intensity over the cross section of the reflected and emitted beam profiles were made on tipped and bare fibers (two in each population) using the set-up of FIGS. 13A and 13B. Light from a 5-milliwatt helium neon laser (632.8 nanometers) was used.

A Coherent Radiation, Inc. Model 210 laser power meter and detector (25 mm aperture) was used for all measurements of the Nd:YAG laser beam power emitted from either the bare or the tipped fibers. A United Detector Technologies silicon diode based 81 optometer detector was used to measure the angular distribution of the emitted helium neon beam (632.8 nanometers) for each fiber type.

Measurements of laser power emitted by groups of nine each bare and tipped fibers were made under identical conditions of 5, 10 and 20 watts input Nd:YAG laser power. A seven centimeter distance between fiber end or tip and detector was maintained for all measurements. Values of average power and standard deviation for both bare and tipped fibers for the three different input Nd:YAG laser powers are found in the following Table 1.

TABLE 1

| Measured Nd:YAG Laser Power Values From Bare and Tipped Fibers | | | |
|---|---|---|---|
| Laser Power Settings (w) | Bare Fiber (w) | Tipped Fiber (w) | Distance (cm) |
| 5 | $4.62 \pm 0.29^{a,b}$ | $3.36 \pm 0.15^{b}$ | 7 |
| 10 | $9.62 \pm 0.34^{b}$ | $7.06 \pm 0.48^{b}$ | 7 |
| 20 | $18.17 \pm 0.68^{b}$ | $13.29 \pm 0.82^{b}$ | 7 |
| 10 | $9.72 \pm 0.32^{c}$ | $8.81 \pm 0.21^{c}$ | 1 |

[a] Numbers are average ± standard deviation
[b] n = 9
[c] n = 3

As seen in Table 1 the bare fibers on the average emitted 92, 96 and 91 percent of the input laser power at 5, 10 and 20 watts. This clustering suggests reproducible light transmission by the parent fiber family.

Results for the nine tipped fibers in Table 1 show the strong uniformity of the percentage of reflected laser power. At laser input power values at 5, 10 and 20 watts, respectively, detected power values from the tipped fibers were nominally 73 percent (see Table 1) of the average power emitted from the bare tip of the parent fiber at each value of input laser power. This large difference arises from the reflected beam diameter exceeding the detector aperture diameter at the 7 cm. detector to tip distance: thus only a fraction of the total reflected power was measured.

In a subsequent experiment, the distance between the detector surface and the tip 12 was decreased to one centimeter. The detected average value of power emitted at 10 watts of laser input from three tipped fibers was 8.8 plus or minus 0.21 watts. Light distribution analysis described below ensured that at this one centimeter detector to reflecting tip surface distance that essentially 100 percent of the reflected light was collected by the detector. Relative to the average emitted power of the bare fiber family (9.72 plus or minus 0.32 watts) at 10 watts input the tipped fiber emits approximately 92 plus percent of the power emitted by the bare tip onto the gold reflecting surface. Allowing for about 98 percent theoretical reflectivity of the pure gold surface and the possibility of two or more multiple reflections from the roughness of a real production gold surface and the non-zero standard deviations associated with the experimentally determined average values of power emitted by both bare and tipped fibers, actual differences between bare fiber and reflected power values can be considered to be negligibly small and the bare fiber and tipped fibers can be considered to emit essentially equal power values at equal Nd:YAG laser input values.

Light distributions from He-Ne neon laser light (632.8 nm.) were determined for representative bare fiber and tipped fibers (two types each). The average distributions are shown in FIG. 14. Examination of these figures shows a spread of the beam from the bare fiber upon reflection in the tip 12. This undoubtedly arises from a small diffuse scattering component due to surface irregularities on the real gold surface. This scattering spreads the beam from the tip 12 in essentially symmetrical fashion about the central ray. The significant effect of reflection of the beam from the fiber end 31 is one of widening without introduction of relatively more or less intense regions of light that is hot or cold spots within the bell-shaped light pattern emitted by the bare tip fiber. Therefore, the shape of the bare fiber beam and that of the reflected side directed beam from tip 12 are essentially equivalent.

Integration of the light flux emitted by both fiber tips over the detector aperture gives the total power incident upon the detector. The flux integral P is given by:

$$P=\frac{2}{DZ}P_0\int_0^{r_0} F(\theta)\,COS^4\theta\,rdr$$

where $P_0$ is the total light power emitted by the tips, F ($\theta$) is the angular variation in the light intensity, and r is the radial distance on the surface of the detector of aperture radius $r_0$. The geometry is shown in FIGS. 12A and 12B. Percentage values of the integral for various detector apertures are shown in FIG. 15 for both bare and tipped fibers. Performance of the integration for the tipped fiber placed 7 and 1 centimeters, respectively, away from the detector surface gives P = 0.717 and 1.0 x the emitted power, respectively. These values are consistent with average values measured at distances of 7 and 1 cm. of, respectively, 0.73 and 0.91 x the average power emitted by the bare fiber upon the reflecting surface 38 of tip 12. Since light back scattering decreases as wavelength increases relative to scatterer sizes going about as one divided by wavelength raised to the nth power (n typically between 1 and 4), the width of the light distribution at the 1.06 micron Nd:YAG wavelength reflected from the tip 12 is expected to be slightly smaller than the measured distribution at 632.8 nanometers.

Values of the fraction of total laser power contained within the beam as angle increases (radial distance increases) are plotted in FIG. 15 for both the bare and tipped fibers. Use of these plots shows that (1 minus exp(-2)) of the total power within the beam from the bare fiber end and the tip 12 is contained within 5 and 16 degrees, respectively, of the central ray of the light distribution. As the result of the wider nature of beam from tip 12 equal spot sizes of radius R. tissue are achieved with the respective bare and tipped fibers held at different distances D. from the tissue surface. For various spot sizes R. in the range of 1 to 4 millimeters, the associated values of the distance to tissue are tabulated in the following Table 2.

TABLE 2

| Spot Size and Distance Values for Bare and Tipped Fibers | | | |
|---|---|---|---|
| $R_0$ (mm) | $\theta_0$ (deg) | D (cm) | Fiber Type |
| 1 | 5 | 1.14 | Bare |
| 1 | 16 | 0.35 | Tipped |
| 2 | 5 | 2.28 | Bare |
| 2 | 16 | 0.70 | Tipped |
| 4 | 5 | 4.56 | Bare |
| 4 | 16 | 1.40 | Tipped |
| Tan $\theta_0$ = $R_0/D_0$ | | | |

The results of optical property assessments on the tip 12 and the bare fibers allow the following essential equivalences to be identified:

(1) Output beams of both tips are centrosymmetric with respect to the central ray of peak intensity with uniform gradual fall off in intensity with radius in all directions. Essentially no random hot or cold spots are present.
(2) The tip 12 reflects essentially all (91 + %) incident laser beam energy. Power densities at tissue equal to that of equivalent bare fiber can be achieved with the use of tip 12 by adjusting the tip to tissue distance, thus adjusting spot size.
(3) The essential action of the tip 12 is to reflect the laser beam laterally.

The substantially complete reflectivity of the gold mirror surface 38 also means that there is no clinically significant heating of the tip 12 itself, i.e., the tip 12 does not become sufficiently hot to significantly affect the tissue being treated if the tip 12 is placed in contact with the tissue but is oriented so that the reflected light beam does not fall upon the tissue contacted by the tip. This is shown by the following example.

EXAMPLE

The depths of coagulation achieved by using the tip 12 as a contact heating probe (no direct irradiation) and by directly irradiating tissue (no direct contact) were compared using 60 W of Nd:YAG laser power. Bovine liver (*in vitro*) was used as the test tissue. The arrangement for each experiment with the tip 12 is shown in FIGS. 16 and 17. The distance of tip 12 from the tissue is 4 mm in FIG. 16 which gives a spot size approximately equal to the 2.2 mm diameter contact size in FIG. 17. In FIG. 6 the tip 12 is immersed in water 206 in a beaker 204. The test tissue 200 is placed against one side of the beaker.

After exposure for one minute with 60 W laser input power, surface bleaching of the liver color was noted at both the contact and irradiated sites. In FIG. 16 the bleached spot is indicated at 202. Perpendicular sections cut with a scalpel through each bleached spot disclosed coagulation bleaching to extend to depth of approximately 700 micron (0.7 mm) and 7 mm at the contacted and irradiated sites, respectively. Thus, direct exposure to the laser beam as opposed to only heating the tip (in contact only with tissue) with the beam resulted in a 10-fold deeper coagulation depth and a 10-fold greater coagulation efficiency at equal exposure times.

## Claims

1. A light reflecting tip apparatus (10) for mounting upon a distal end portion (14) of a flexible elongated light transmitting fiber (16), comprising:

   an elongated cylindrical body (18) having a fiber receiving longitudinal opening (30) defined therein for receiving said distal end portion (14) of said fiber (16), and having a lateral passageway (88);

   a reflecting mirror surface (38) defined in said body (18) for reflecting light from said fiber (16) laterally outward through said lateral passageway (88); characterised in that said lateral passageway (88) is defined diametrically through said body (18) and intersecting said longitudinal opening (30), said lateral passageway (88) having diametrically opposite first and second end openings (90, 92);

   and in that said lateral passageway (88) provides a flushing means for permitting surrounding fluid to flow in one of said first and second end openings (90, 92), then across said reflecting mirror surface (38), then out the other of said first and second end openings (90, 92) to reduce collection of contaminants on said reflecting mirror surface (38).

2. An operating assembly according to claim 1, characterised in that said reflecting mirror surface (38) is constructed to reflect substantially all light falling thereon.

3. An operating assembly according to claim 1 or 2, characterised in that there is an axial clearance between the distal fibre end (31) and the mirror surface (38) to permit all of the reflected beam to exit laterally without impinging on the fibre end (31).

4. An operating assembly according to claim 1, characterised in that said flushing passageway end openings (90,92) are laterally on opposite sides of said reflecting surface (38).

5. An operating assembly according to claim 4, characterised in that said tip (12) is generally cylindrical in shape.

6. An operating assembly according to any preceding claim, characterised in that said tip (12) is free from attachment to any structure other than said fibre (16).

7. An operating assembly according to any preceding claim, characterised in that said fibre (16) includes a core (22), a cladding surrounding said core (22), and an outer protective jacket surrounding said cladding; said tip including a hollow crimping cylinder (68) extending proximally therefrom; said tip (12) and said hollow crimping shoulder (68) having a tip body bore (30) defined therein from a proximal end of said hollow crimping cylinder (68), said distal end portion (14) of said fibre (16) being received in said tip body bore (30); and said tip (12) including at least one crimp (78,80) in said crimping cylinder (68), said crimp (78,80) mechanically attaching said tip (12) to said outer protective jacket of said fibre (16).

8. An operating assembly according to any preceding claim, characterised in that said reflecting mirror surface (38) is made of gold.

9. An operating assembly according to claim 8, characterised in that a body (18) of said tip is solid gold.

10. An operating assembly according to claim 8, characterised in that gold reflecting mirror surface (38) is coined.

**Patentansprüche**

1. Eine Vorrichtung (10) mit lichtreflektierender Spitze zum Anbringen an einem distalen Endbereich (14) einer flexiblen, langgestreckten Lichtübertragungsfaser (16), aufweisend:

   einen langgestreckten, zylindrischen Körper (18) mit einer die Faser aufnehmenden, in Längsrichtung verlaufenden Öffnung (30), die hierin zum Aufnehmen des genannten distalen Endbereichs (14) der genannten Faser (16) definiert ist, und mit einem seitlichen Durchgang (88);

   eine reflektierende Spiegelfläche (38), die in dem genannten Körper (18) zum Reflektieren von Licht von der genannten Faser (16) seitlich nach auswärts durch den genannten seitlichen Durchgang (88) definiert ist; **dadurch gekennzeichnet**, daß der genannte seitliche Durchgang (88) diametral durch den genannten Körper (18) definiert ist und sich mit der genannten, in Langsrichtung verlaufenden Öffnung (30) kreuzt, wobei der genannte seitliche Durchgang (88) eine erste Endöffnung (90) und eine zweite Endöffnung (92) aufweist und sich die beiden Endöffnungen (90, 92) diametral gegenüberliegen, und daß der genannte seitliche Durchgang (88) eine Spüleinrichtung vorsieht, um es einem umgebenden Fluid zu erlauben, in eine der Endöffnungen (90, 92), nämlich erste Endöffnung (90) und zweite Endöffnung (92), sodann quer über die genannte reflektierende Spiegelfläche (38) und sodann aus der anderen der genannten Endöffnungen (90, 92), nämlich erste Endöffnung (90) und zweite Endöffnung (92), heraus zu strömen, um eine Ansammlung von Verunreinigungen an der genannten reflektierenden Spiegelfläche (38) zu reduzieren.

2. Eine Betriebsanordnung nach Anspruch 1, **dadurch gekennzeichnet**, daß die genannte reflektierende Spiegelfläche (38) so ausgebildet ist, um im wesentlichen sämtliches darauf fallendes Licht zu reflektieren.

3. Eine Betriebsanordnung nach Anspruch 1 oder 2, **dadurch gekennzeichnet**, daß es ein axiales Spiel zwischen dem distalen Faserende (31) und der Spiegelfläche (38) gibt, um es dem gesamten reflektierten Strahl zu erlauben, seitlich ohne Auftreffen auf das Faserende (31) auszutreten.

4. Eine Betriebsanordnung nach Anspruch 1, **dadurch gekennzeichnet**, daß sich die genannten Spüldurchgangs-Endöffnungen (90, 92) seitlich an gegenüberliegenden Seiten der genannten reflektierenden Fläche (38) befinden.

5. Eine Betriebsanordnung nach Anspruch 4, **dadurch gekennzeichnet**, daß die genannte Spitze (12) im wesentlichen zylindrisch in der Gestalt ist.

6. Eine Betriebsanordnung nach einem der vorhergehenden Ansprüche, **dadurch gekennzeichnet**, daß die genannte Spitze (12) von einer Anbringung an irgendeine andere Struktur als die genannte Faser (16) unabhängig ist.

7. Eine Betriebsanordnung nach einem der vorhergehenden Ansprüche, **dadurch gekennzeichne**t, daß die genannte Faser (16) einen Kern (22), eine den genannten Kern (22) umgebende Hülle und einen äußeren Schutzmantel aufweist, welcher die genannte Hülle umgibt, wobei die genannte Spitze einen hohlen Einbördelungszylinder (68) aufweist, der sich von dieser proximal erstreckt, wobei die genannte Spitze (12) und die genannte hohle Einbördelungsschulter (68) eine Spitzenkörperöffnung (30) aufweisen, welche hierin von einem proximalen Ende des genannten hohlen Einbördelungszylinders (68) definiert ist, wobei der genannte distale Endbereich (14) der genannten Faser (16) in der genannten Spitzenkörperöffnung (30) aufgenommen ist, wobei die genannte Spitze (12) wenigstens eine Einbördelung (78, 80) bei dem genannten Einbördelungszylinder (68) aufweist und wobei die genannte Einbördelung (78, 80) die genannte Spitze (12) an dem genannten äußeren Schutzmantel der genannten Faser (16) mechanisch befestigt.

8. Eine Betriebsanordnung nach einem der vorhergehenden Ansprüche, **dadurch gekennzeichnet,** daß die genannte reflektierende Spiegelfläche (38) aus Gold hergestellt ist.

9. Eine Betriebsanordnung nach Anspruch 8, **dadurch gekennzeichnet**, daß ein Körper (18) der genannten Spitze massives Gold ist.

10. Eine Betriebsanordnung nach Anspruch 8, **dadurch gekennzeichnet**, daß die aus Gold bestehende, reflektierende Spiegelfläche (38) geprägt ist.

**Revendications**

1.  Ensemble opérationnel (10) à embout réfléchissant la lumière, destiné à être monté sur une portion d'extrémité distale (14) d'une fibre allongée flexible (16) transmettant la lumière, comprenant :

    - un corps cylindrique allongé (18) ayant une ouverture longitudinale (30) recevant la fibre, définie dans le corps, pour recevoir ladite portion d'extrémité distale (14) de ladite fibre (16), et ayant un passage latéral (88) ;
    - une surface de miroir réflecteur (38) définie dans ledit corps (18) pour réfléchir une lumière en provenance de ladite fibre (16) latéralement vers l'extérieur à travers ledit passage latéral (88),
    caractérisé en ce que ledit passage latéral (88) est défini diamétralement à travers ledit corps (18) et croise ladite ouverture longitudinale (30), ledit passage latéral (88) ayant des première et deuxième ouvertures terminales (90, 92) diamétralement opposées, et en ce que ledit passage latéral (88) procure un moyen de rinçage pour permettre à un fluide environnant de passer dans l'une desdites première et deuxième ouvertures terminales (90, 92), puis le long de ladite surface de miroir réflecteur (38), puis de sortir de l'autre desdites première et deuxième ouvertures terminales pour réduire l'accumulation de contaminants sur ladite surface de miroir réflecteur.

2.  Ensemble opérationnel selon la revendication 1, caractérisé en ce que ladite surface de miroir réflecteur (38) est construite pour réfléchir pratiquement toute la lumière tombant sur elle.

3.  Ensemble opérationnel selon la revendication 1 ou 2, caractérisé en ce qu'il existe un intervalle axial entre l'extrémité distale (31) de la fibre et la surface de miroir (38) pour permettre à la totalité du faisceau réfléchi de sortir latéralement sans tomber sur l'extrémité (31) de la fibre.

4.  Ensemble opérationnel selon la revendication 1, caractérisé en ce que lesdites ouvertures terminales (90, 92) du passage de rinçage se situent latéralement sur des côtés opposés de ladite surface réfléchissante (38).

5.  Ensemble opérationnel selon la revendication 4, caractérisé en ce que ledit embout (12) a une forme généralement cylindrique.

6.  Ensemble opérationnel selon l'une des revendications précédentes, caractérisé en ce que ledit embout (12) n'est attaché sur aucune autre structure que ladite fibre (16).

7.  Ensemble opérationnel selon l'une des revendications précédentes, caractérisé en ce que ladite fibre (16) comprend une âme (22), une gaine entourant ladite âme (22) et une chemise protectrice extérieure entourant ladite gaine, ledit embout comprenant un cylindre de sertissage creux (68) s'en étendant de son extrémité proximale, un alésage de corps d'embout (30) étant défini à l'intérieur dudit embout (12) dudit cylindre de sertissage creux (68) depuis l'extrémité proximale dudit cylindre de sertissage creux (68), ladite portion d'extrémité distale (14) de ladite fibre (16) étant logée dans ledit alésage de corps d'embout (30) ; et ledit embout (12) comprenant au moins un sertissage (78, 80) dans ledit cylindre de sertissage (68) fixant mécaniquement l'embout (12) dans ladite chemise protectrice extérieure de ladite fibre (16).

8.  Ensemble opérationnel selon l'une des revendications précédentes, caractérisé en ce que ladite surface de miroir réflecteur (38) est en or.

9.  Ensemble opérationnel selon la revendication 8, caractérisé en ce que le corps (18) dudit embout est en or massif.

10. Ensemble opérationnel selon la revendication 8, caractérisé en ce que la surface de miroir réflecteur (38) est emboutie.

FIG. 1

FIG. 2

FIG. 3

FIG. 4

FIG. 5

FIG. 6

13

FIG. 7

FIG. 8

FIG. 9

14

12

16

*SEE FIG. 11*

118

120

12

120

16

FIG. 11

116

117

108

LASER

16

16

16

FIG. 10

15

CENTRAL RAY

d
r
R
$r_0$

POWER
METER
DETECTOR

16

## FIG. 12A

CENTRAL RAY

112°

d
r
R
$r_0$

POWER
METER
DETECTOR

12

## FIG. 12B

**FIG. 13A**

**FIG. 13B**

ANGULAR LIGHT INTENSITY DISTRIBUTION OF REPRESENTATIVE BARE
AND TIPPED OPTICAL FIBER MEASURED AT 632.8 NM WAVELENGTH.

FIG. 14

PERCENTAGE OF TOTAL EMITTED LASER POWER VERSUS INCLUDED
ANGLE (θ) FOR REPRESENTATIVE BARE AND TIPPED FIBERS
AT 632.8 NM.

FIG. 15

FIG. 16

FIG. 17